# EUROPEAN PATENT APPLICATION

(11) **EP 2 163 249 A1**
(43) Date of publication of application: **17.03.2010**
(21) Application number: 08764571.9
(22) Date of filing: 23.05.2008
(51) Int. Cl.: A61K 31/7004, A61K 9/08, A61K 31/19, A61K 31/191, A61K 31/198, A61K 31/51, A61K 47/02, A61K 47/12, A61K 47/20, A61K 47/22, A61K 47/26, A61P 3/02

(54) **INFUSION SOLUTION FOR PERIPHERAL INTRAVENOUS ADMINISTRATION**

(30) Priority: 25.05.2007 JP 2007138604
(71) Applicant: Ajinomoto Co., Inc., Tokyo 104-8315 (JP)
(72) Inventor: SHIGETA, Mutsuo, Shizuoka-shi Shizuoka 424-0911 (JP); ABIKO, Kazuhiro, Shizuoka-shi Shizuoka 424-0911 (JP)
(74) Representative: Nicholls, Kathryn Margaret
(86) International application number: PCT/JP2008/059525
(87) International publication number: WO 2008/146732

(57) **Abstract**

There is provided an infusion solution for peripheral intravenous administration comprising a multichamber vessel constructed in a connectable manner, which separately houses a sugar solution containing sugars, electrolytes and vitamin B1, and an amino acid solution containing amino acids, electrolytes and a sulfite, whereby the vitamin B1 and amino acids are stably maintained.

In the infusion solution for peripheral intravenous administration, the sugar solution contains calcium gluconate, contains no greater than 1.4 g/L sodium lactate, has a pH of 4.0-4.7 and has a titratable acidity of 2-4, the amino acid solution contains 4.0-8.0 g/L sodium lactate, contains no calcium gluconate, has a pH of 6.8-7.2, contains free L-cysteine as cysteine and contains 0.05-0.2 g/L sodium hydrogen sulfite, and after the sugar solution and amino acid solution have been mixed, the infusion solution has a pH of 6.5-7.1 and a titratable acidity of 5-10.

## Description

### TECHNICAL FIELD

The present invention relates to an infusion solution for peripheral intravenous administration to be used as nutritional supplementation, comprising a multichamber vessel constructed in a connectable manner, which separately houses a sugar solution containing a sugar, electrolytes and vitamin B1, and an amino acid solution containing amino acids, electrolytes and a sulfite.

### BACKGROUND ART

Infusions for nutritional supplementation are largely classified as either infusion solutions for central intravenous administration whereby large amounts of nutrients are supplied to large volumes of venous blood through the central vein, and infusion solutions for peripheral intravenous administration whereby relatively small amounts of nutrients are supplied through peripheral veins.
Infusions for central intravenous administration are intravenous hyperalimentation solutions containing saccharides, electrolytes and amino acids, and they are used in cases of prolonged undernourishment or cases which require nutritional management for two weeks or longer during highly invasive procedures, being injected into the large volume of venous blood in the central vein.
Infusions for peripheral intravenous administration, on the other hand, are administered for relatively brief periods through the antebrachial vein using peripheral indwelling needles, with the aim of supporting or improving the nutritional status of patients that are undergoing noninvasive or lightly invasive procedures and that have a relatively good nutritional status.

All of the components of an infusion are preferably housed in a single container, from the viewpoint of convenience of use and preventing errors during administration. However, since mixtures of amino acids and glucose in saccharide components undergo alteration by Maillard reaction, acidified mixtures with pH of about 5 have been developed to prevent this. Injection of an infusion solution for central intravenous administration into large-volume venous blood presents no problem even with such a low pH. With infusion solutions for peripheral intravenous administration, however, a low pH often causes phlebitis and angialgia. For this reason, a sugar solution is housed in one chamber of a two-chamber container together with one portion of the electrolytes, and amino acids are separately housed in the other chamber together with another portion of the electrolytes, in order to maintain the shelf life of the formulation while ensuring that the pH of the mixed infusion is near the physiological pH of blood.

Vitamin B1 supplementation is known to be essential in the case of intravenous hyperalimentation administered through the central vein, and this has been accomplished by different modes (Patent documents 1 and 2). However, since vitamin B1 is consumed during metabolism of intravenously administered sugars even when using infusions for peripheral intravenous administration, infusions for peripheral intravenous administration that contain added vitamin B1 are being marketed, such as PARESAFE^{™}, AMIGRAND^{™} or BFLUID^{™}.

Vitamin B1 is stable in under acidic conditions but unstable under neutral-alkaline conditions. The pH range for thiamine parenteral solutions is set to 2.5-4.5, for example, according to the Japanese Pharmacopeia. In addition, with an infusion solution for peripheral intravenous administration where a sugar solution is housed together with one portion of the electrolytes and vitamin B1 in one chamber of a two-chamber container while amino acids and another portion of the electrolytes are separately housed in the other chamber, the sulfite that is used as a stabilizer for the amino acids destabilizes the vitamin B1 in the infusion after the sugar solution and amino acid solution have been mixed, and it is therefore used in a small amount.

However, since a low pH during administration of an infusion can cause phlebitis, it is necessary to keep the pH of the administered infusion as close to neutral as possible, to limit the total amount of pH-adjusting acids such as acetic acid added to the sugar solution and amino acid solution, to no greater than a specified amount, and to maintain the titratable acidity of the mixed infusion to no higher than 10 and preferably 5-10. Therefore, a sugar solution to which vitamin B1 is to be added cannot have its pH excessively low, and it is normally pH 4-5.

When the pH of the sugar solution is adjusted to 4-5 for stabilization of vitamin B1, the pH of the amino acid solution must be higher than that of the sugar solution in order to ensure that the pH of the mixed infusion is neutral. If the amino acid solution pH is too high, however, the amino acids such as L-cysteine become unstable and a large amount of sulfite must therefore be added as an amino acid stabilizer. Yet using a large amount of a sulfite is undesirable since it destabilizes vitamin B1 in the infusion containing the mixture of the sugar solution and amino acid solution. Furthermore, when the pH of the amino acid solution is adjusted, it is essential to maintain the total amount of pH-adjusting acids including acetic acid which are added to the sugar solution and amino acid solution, to no greater than fixed amount, and to limit the titratable acidity of the mixed infusion to no greater than 10. Much research has been conducted on compositions of infusions for peripheral intravenous administration in two-chamber containers, where a vitamin B1-added sugar solution is placed in one chamber and an amino acid solution is added to the other chamber (Patent documents 3 and 4).

For an infusion solution for peripheral intravenous administration, it is preferred to limit the amount of chloride ions from the electrolytes in order to prevent hyperchloremic acidosis. Therefore, instead of using sodium chloride as the entire sodium source, a portion is added as sodium lactate. When using commercially available PARESAFE^{™}, glucose is added as a sugar and sodium chloride, sodium lactate, calcium gluconate, magnesium sulfate and zinc sulfate are added as electrolytes to the sugar solution to which vitamin B1 is to be added. In order to ensure the stability of the vitamin B1 in the sugar solution the pH must be set to 3.8-4.8, and therefore a large amount of acetic acid must be added to the sugar solution as a pH regulator. On the other hand, since a low pH of the infusion after the sugar solution and amino acid solution have been mixed can lead to phlebitis, the pH of the mixed infusion is desirably near neutral and the titratable acidity is no greater than 10. In other words, it is essential to keep the total amount of acetic acid, which is used as the pH regulator, to no greater than a fixed amount in the infusion after the sugar solution and amino acid solution have been mixed, and thus when a large amount of acetic acid is used to lower the pH of the sugar solution, the amount of acetic acid that can be added to the amino acid solution is necessarily reduced.

In the case of commercially available PARESAFE^{™}, vitamin B1 is added to 350 mL of electrolyte-containing sugar solution to pH 4.3, with acetic acid being added at approximately 7 mEq and the titratable acidity at 20. The 350 mL of sugar solution is mixed with 150 mL of amino acid solution. In order to limit the titratable acidity of the infusion to no greater than 10 when these two solutions are mixed, it is necessary for the amount of acetic acid to be kept at about 9.5 mEq in 500 mL of infusion. The amount of acetic acid that can be added to the amino acid solution, therefore, is approximately 2.5 mEq, and the pH of the amino acid solution will be approximately 8 after adjustment of the pH with acetic acid. However, an amino acid solution pH of around 8 will destabilize the active ingredient L-cysteine, and there is a risk of its content being reduced especially during heat sterilization.
[Patent document 1] Japanese Unexamined Patent Publication HEI No. 8-143459
[Patent document 2] Japanese Unexamined Patent Publication HEI No. 9-59150
[Patent document 3] Japanese Unexamined Patent Publication No. 2003-55195
[Patent document 4] WO2004/103375

### DISCLOSURE OF INVENTION

### [Problems to be Solved by the Invention]

It is an object of the present invention to provide an infusion solution for peripheral intravenous administration in a multichamber vessel housing a sugar solution containing vitamin B1 in one chamber of the multichamber vessel and housing amino acids in another chamber, which is an infusion solution for peripheral intravenous administration in a two-chamber container wherein the pH of the sugar solution is lowered with a relatively small amount of acid to maintain the stability of the vitamin B1, and the pH of the amino acid solution is adjusted to near neutral with a small amount of a sulfite to stabilize the amino acids such as L-cysteine, so that the infusion is at near neutral after mixing the sugar solution and amino acid solution.

### [Means for Solving the Problems]

In the course of devising the present invention it was discovered that addition of calcium gluconate to the sugar solution and addition of sodium lactate to the amino acid solution can produce a pH which allows the vitamin B1 to stably exist in the sugar solution with a small amount of acid, while also adjusting the pH of the amino acid solution to around 7 to improve the stability of L-cysteine, reducing sulfites as stabilizers and maintaining the infusion to near neutral after mixing.

Commercially available PARESAFE^{™} contains sodium chloride, sodium lactate, calcium gluconate, magnesium sulfate and zinc sulfate added as electrolytes in addition to thiamine hydrochloride, in 350 mL of sugar solution. It also contains dipotassium phosphate as an electrolyte and sodium hydrogen sulfite as an amino acid stabilizer in addition to amino acids, in 150 mL of amino acid solution. When most or all of the sodium lactate added to the sugar solution with this formulation is transferred to the amino acid solution, it was found, surprisingly, that (1) it is possible to adjust the titratable acidity of the sugar solution to 2-4 and the pH to 4.0-4.7 in which range vitamin B1 is stable, while also adjusting the pH of the amino acid solution to neutral (pH 6.8-7.2) at which amino acids are stable, even with the same total amount of acid added to both the sugar solution and the amino acid solution, and that (2) this drastically reduces the amount of sodium hydrogen sulfite stabilizer required to be added to the amino acid solution. With PARESAFE^{R}, when the total amount of acetic acid added to both the sugar solution and amino acid solution was 9.5 mEq, adjusting the pH of the sugar solution to 4.3 resulted in a titratable acidity of 20 for the sugar solution, and the pH of the amino acid solution was approximately 8. However, by transferring all or most of the sodium lactate from the sugar solution to the amino acid solution it was possible to adjust the titratable acidity of the sugar solution to the range of 2-4 and its pH to 4.0-4.7 and to adjust the amino acid solution pH to neutral, even though the amount of acetic acid addition was the same. This was completely unpredictable. As a result of adjusting the amino acid solution pH to near neutral, it was possible to limit the amount of sodium hydrogen sulfite addition required for amino acid stabilization during heat sterilization to 7.5-30 mg per 150 mL of amino acid solution (0.05-0.2 g per 1 L of amino acid solution). That is, the amount of sodium hydrogen sulfite added could be reduced compared to PARESAFE^{R}, wherein 45 mg of sodium hydrogen sulfite (0.3 g per 1 L of amino acid solution) is added because of the amino acid solution pH of approximately 8. Because vitamin B1 is unstable in the infusion prepared by mixing the sugar solution and amino acid solution, reducing the sodium hydrogen sulfite used as the amino acid stabilizer has a major effect for preventing decomposition of vitamin B1 in the infusion.

The invention therefore provides an infusion solution for peripheral intravenous administration comprising a sugar solution containing sugars, a portion of electrolytes and vitamin B1, and an amino acid solution containing amino acids, a portion of electrolytes and a sulfite, separately housed in a multichamber vessel with a connectable construction, wherein (1) the sugar solution contains calcium gluconate, contains no greater than 1.4 g/L sodium lactate, has a pH of 4.0-4.7 and has a titratable acidity of 2-4, (2) the amino acid solution contains 4-15 g/L sodium lactate, contains no calcium gluconate, has a pH of 6.8-7.2, contains free L-cysteine as cysteine and contains 0.05-0.2 g/L sodium hydrogen sulfite, and (3) after the sugar solution and amino acid solution have been mixed, the infusion solution for peripheral intravenous administration has an infusion pH of 6.5-7.1 and a titratable acidity of 5-10.

The following (1)-(5) are preferred modes of the infusion solution for peripheral intravenous administration according to the invention.
(1) An infusion solution for peripheral intravenous administration wherein the sugar solution contains calcium gluconate, contains no sodium lactate, has a pH of 4.0-4.5 and has a titratable acidity of 2-4, the amino acid solution contains sodium lactate, contains no calcium gluconate, has a pH of 6.8-7.2, contains free L-cysteine as cysteine and contains 0.05-0.2 g/L sodium hydrogen sulfite, and after the sugar solution and amino acid solution have been mixed, the infusion solution has a pH of 6.5-7.1 and a titratable acidity of 5-10.
(2) An infusion solution for peripheral intravenous administration wherein the sugar solution contains 50-200 g/L glucose, 1-5 mg/L vitamin B1 as thiamine, 0.5-2 g/L sodium chloride, 1-3 g/L calcium gluconate, 0.0-1.4 g/L sodium lactate, 0.5-2 g/L magnesium sulfate hydrate and 1-4 mg/L zinc sulfate, and the amino acid solution contains 50-200 g/L amino acids as free amino acids, 4-15 g/L sodium lactate, 3-12 g/L dipotassium phosphate and 0.05-0.2 g/L sodium hydrogen sulfite, and the volume ratio of the sugar solution and the amino acid solution is 2-3:1.
(3) An infusion solution for peripheral intravenous administration, wherein the amino acid composition of the amino acid solution, as free amino acids, is as follows: 10-20 g/L L-leucine, 6-12 g/L L-isoleucine, 6-12 g/L L-valine, 8-16 g/L L-lysine, 2-10 g/L L-threonine, 1-3 g/L L-tryptophan, 2-6 g/L L-methionine, 0.5-2 g/L L-cysteine, 4-10 g/L L-phenylalanine, 0.3-1 g/L L-tyrosine, 7-16 g/L L-arginine, 3-8 g/L L-histidine, 5-12 g/L L-alanine, 3-8 g/L L-proline, 2-5 g/L L-serine, 4-9 g/L glycine, 0.5-2 g/L L-aspartic acid, 0.5-2 g/L L-glutamic acid.
(4) An infusion solution for peripheral intravenous administration, wherein the electrolyte composition of the sugar solution is Na⁺: 9-47 mEq/L, Ca²⁺: 5-20 mEq/L, Mg²⁺: 5-20 mEq/L, Zn²⁺: 5-20 µmol/L, Cl⁻: 5-35 mEq/L, the electrolyte composition of the amino acid solution is Na⁺: 35-150 mEq/L, K⁺: 30-140 mEq/L, phosphorus: 15-70 mmol/L, and the volume ratio of the sugar solution and amino acid solution is 2-3:1.
(5) An infusion solution for peripheral intravenous administration, wherein i) to the sugar solution there are added 50-200 g/L glucose, 1-5 mg/L vitamin B1 as thiamine, 0.5-2 g/L sodium chloride, 0.0-1.4 g/L sodium lactate, 1-3 g/L calcium gluconate, 0.5-2 g/L magnesium sulfate hydrate and 1-4 mg/L zinc sulfate, the pH of the sugar solution is 4.0-4.5 and the titratable acidity of the sugar solution is 2-4, (ii) to the amino acid solution there are added, as free amino acids, 10-20 g/L L-leucine, 6-12 g/L L-isoleucine, 6-12 g/L L-valine, 8-16 g/L L-lysine, 2-10 g/L L-threonine, 1-3 g/L L-tryptophan, 2-6 g/L L-methionine, 0.5-2 g/L L-cysteine, 4-10 g/L L-phenylalanine, 0.3-1 g/L L-tyrosine, 7-16 g/L L-arginine, 3-8 g/L L-histidine, 5-12 g/L L-alanine, 3-8 g/L L-proline, 2-5 g/L L-serine, 4-9 g/L glycine, 0.5-2 g/L L-aspartic acid and 0.5-2 g/L L-glutamic acid, with 4-15 g/L sodium lactate, 3-12 g/L dipotassium phosphate and 0.05-0.2 g/L sodium hydrogen sulfite and with L-cysteine being added as free L-cysteine, and the pH of the amino acid solution is 6.8-7.2, and (iii) the volume ratio of the sugar solution and amino acid solution is 7:3, and after the sugar solution and amino acid solution have been mixed, the pH of the infusion is 6.5-7.1.

### [Effect of the Invention]

According to the infusion solution for peripheral intravenous administration of the invention it is possible, with a small amount of acid, to keep the sugar solution at pH 4.0-4.7 which is the pH range in which vitamin B1 is stable and, with a small amount of sodium hydrogen sulfite, to keep the amino acid solution at near pH 7 to allow stabilization of amino acids.

### DETAILED DESCRIPTION OF THE INVENTION

The infusion solution for peripheral intravenous administration according to the invention will now be explained in detail. The gist of the invention lies in the addition of calcium gluconate to the sugar solution and addition of all or most of the sodium lactate to the amino acid solution. The invention may employ hitherto publicly known techniques and modifications based thereon, within the scope of the gist thereof.

### <Sugar solution>

The sugar solution in the infusion solution for intravenous administration according to the invention is composed of fundamentally sugars, electrolytes and vitamin B1, and contains no sulfites that might interfere with stabilization of the vitamin B1. The sugars to be used are not particularly restricted so long as they are sugars ordinarily incorporated in infusions, and for example, there may be mentioned reducing sugars such as glucose, fructose and maltose, and non-reducing sugars such as trehalose, xylitol, sorbitol and glycerin. Of the sugars mentioned above, glucose is preferably added from the viewpoint of a nutritive effect. When using glucose, the glucose is used at a concentration of 35-150 g/L, preferably 70-130 g/L and most preferably 90-120 g/L in the sugar solution. The pH of the sugar solution is pH 4.0-4.7, preferably 4.0-4.5 and most preferably pH 4.3-4.5 sugar solution for stable addition of vitamin B1. The pH regulator used is preferably using glacial acetic acid.
The vitamin B1 added to the sugar solution may be any publicly available vitamin B1, of which thiamine hydrochloride, thiamine nitrate and fursultiamine may be mentioned as examples. Thiamine hydrochloride is particularly preferred as vitamin B1. The amount of vitamin B1 is preferably 2-3.5 mg/L in the sugar solution.
The electrolytes added to the sugar solution are preferably sodium chloride, calcium gluconate, sodium lactate, magnesium sulfate and zinc sulfate. In the case of sodium chloride, it is preferably added at 0.5-2 g/L and most preferably 1.14 g/L. In the case of calcium gluconate, it is preferably added at 1-3 g/L and most preferably 1.6 g/L. In the case of sodium lactate, it is preferably added at no greater than 1.4 g/L, and most preferably it is not added. In the case of magnesium sulfate hydrate, it is preferably added at 0.5-2 g/L and most preferably 0.89 g/L. In the case of zinc sulfate, it is preferably added at 1-4 mg/L and most preferably 2 mg/L.

### <Amino acid solution>

The amino acid solution comprises amino acids including at least essential amino acids, and the amino acids are added at 50-200 g/L, preferably 75-150/L and most preferably 100 g/L as free amino acids. The amino acids may also be in forms other than free amino acids, such as various salts including salts of metals such as sodium and potassium, salts of organic acids such as acetic acid and salts of inorganic acids such as hydrochloric acid. A portion thereof may also be in acylated or peptide form, although free amino acids or their salts are preferred from the viewpoint of nutritional supplementation. L-cysteine, in particular, is unstable and may therefore be added in N-acetylated form from the viewpoint of preventing decomposition, but since metabolism will be required to yield free L-cysteine, free L-cysteine should be added from the viewpoint of nutritional supplementation.

The amino acid composition of the amino acid solution may be 10-20 g/L and most preferably 14 g/L L-leucine, 6-12 g/L and most preferably 8 g/L L-isoleucine, 6-12 g/L and most preferably 8 g/L L-valine, 8-16 g/L and most preferably 10.49 g/L L-lysine, 2-10 g/L and most preferably 5.7 g/L L-threonine, 1-3 g/L and most preferably 2 g/L L-tryptophan, 2-6 g/L and most preferably 3.9 g/L L-methionine, 0.5-2 g/L and most preferably 1 g/L L-cysteine, 4-10 g/L and most preferably 7 g/L L-phenylalanine, 0.3-1 g/L and most preferably 0.5 g/L L-tyrosine, 7-16 g/L and most preferably 10.5 g/L L-arginine, 3-8 g/L and most preferably 5 g/L L-histidine, 5-12 g/L and most preferably 8 g/L L-alanine, 3-8 g/L and most preferably 5 g/L L-proline, 2-5 g/L and most preferably 3 g/L L-serine, 4-9 g/L and most preferably 5.9 g/L glycine, 0.5-2 g/L and most preferably 1 g/L L-aspartic acid, and 0.5-2 g/L and most preferably 1 g/L L-glutamic acid, as free amino acids.

Sodium lactate and dipotassium phosphate are preferably added as electrolytes to the amino acid solution. In the case of sodium lactate, it is preferably added at 4-15 g/L and most preferably 7.6 g/L. In the case of dipotassium phosphate, it is added at 3-12 g/L and most preferably 5.8 g/L.

Sodium hydrogen sulfite is added at 0.05-0.2 g/L to the amino acid solution for stabilization of the amino acids. Even if sodium hydrogen sulfite is added at greater than 0.2 g/L, the amino acid-stabilizing effect will not be significantly affected with the sugar solution and amino acid solution formulation specified above. On the other hand, since the sulfite destabilizes vitamin B1 when the sugar solution and amino acid solution are mixed, a range of 0.05-0.2 g/L was selected as the optimal range for amino acid stabilization.

### <Other components>

Various vitamins and trace elements may also be added as desired or necessary.

### <Infusion comprising mixture of sugar solution and amino acid solution>

The sugar solution and amino acid solution are mixed to produce the infusion when the connecting partition is removed by external pressing force at the time of use. The infusion solution is for peripheral intravenous injection, and it preferably has a pH of 6.5-7.1 and a titratable acidity in the range of 5-10 in order to prevent phlebitis and angialgia during administration. The volume ratio of the sugar solution and amino acid solution is 2-3:1 and most preferably 7:3.
The amounts of electrolytes in the infusion are follows: Na⁺: 17.5-70 mEq/L and preferably 35 mEq/L, K⁺: 10-40 mEq/L and preferably 20 mEq/L, Mg²⁺: 2.5-10 mEq/L and preferably 5 mEq/L, Ca²⁺: 2.5-10 mEq/L and preferably 5 mEq, Cl⁻: 17.6-70.4 mEq/L and preferably 35.2 mEq/L, Zn²⁺: 2.4-9.6 µmol/L and preferably 4.8 µmol/L, phosphorus: 5-20 mmol/L and preferably 10 mmol/L.

### <Infusion container>

Any publicly known multichamber vessel that is constructed in a connectable manner may be used. Infusion bags constructed with partitions or readily-releasable seals are especially preferred for convenient connection. The material of the infusion bag may be, for example, a polyolefin such as polyethylene, polypropylene or polybutene, ethylene/propylene copolymer, crosslinked ethylene/vinyl acetate copolymer, or laminates thereof.

### <Exterior packaging and iron-based deoxidizer>

The infusion bag filled with the sugar solution and amino acid solution is packaged, together with the deoxidizer, using a light-resistant gas impermeable outer packaging material by an ordinary method. The light-resistant gas impermeable outer packaging material may be a commonly used aluminum foil or aluminum vapor deposition film. When a transparent gas-impermeable outer packaging material is selected, such as polyethylene terephthalate, polyethylene naphthalate, ethylene/vinyl alcohol copolymer, polyvinylidene chloride, polyacrylonitrile, polyamide, an alumina vapor deposition film or a silica vapor deposition film, it may be used after adding an ultraviolet ray-blocking layer.
Oxygen absorbers composed mainly of iron compounds such as iron hydroxide, iron oxide and iron carbide may be used. As commercially available products there may be mentioned AGELESS^{™} (Mitsubishi Gas Chemical Co., Inc.), MODULAN^{™} (Nippon Kayaku Co., Ltd.) and SEQUL^{™} (Nippon Soda Co., Ltd.).
The space between the infusion bag and the exterior packaging container is preferably filled with an inert gas such as nitrogen.

### EXAMPLES

Examples of the invention will now be described.

### [Example 1]

| <Formulation of amino acid solution> | |
|---|---|
| L-Leucine | 14 g/L |
| L-Isoleucine | 8 g/L |
| L-Valine | 8 g/L |
| L-Lysine hydrochloride | 13.1 g/L |
| L-Threonine | 5.7 g/L |
| L-Tryptophan | 2 g/L |
| L-Methionine | 3.9 g/L |
| L-Cysteine | 1.1 g/L |
| L-Phenylalanine | 7 g/L |
| L-Tyrosine | 0.5 g/L |
| L-Arginine | 10.5 g/L |
| L-Histidine | 5 g/L |
| L-Alanine | 8 g/L |
| L-Proline | 5 g/L |
| L-Serine | 3 g/L |
| Glycine | 5.9 g/L |
| L-Aspartic acid | 1 g/L |
| L-Glutamic acid | 1 g/L |
| Sodium lactate solution, 50% | 15.3 g/L |
| Dipotassium phosphate | 5.8 g/L |
| Sodium hydrogen sulfite | 0.05 g/L |

The formulation of amino acids and electrolyte components listed above was dissolved in water for injection, and after adjusting to pH 7.0 using glacial acetic acid as the pH regulator, sodium hydrogen sulfite was dissolved therein.

| <Sugar solution formulation> | |
|---|---|
| Glucose | 107 g/L |
| Sodium chloride | 1.14 g/L |
| Calcium gluconate | 1.6 g/L |
| Magnesium sulfate hydrate | 0.89 g/L |
| Zinc sulfate | 2 mg/L |
| Thiamine hydrochloride | 2.86 mg/L |

The formulation of sugar, electrolytes and vitamin B1 listed above was dissolved in water for injection, and glacial acetic acid was used as a pH regulator to pH 4.3.
Both solutions were aseptically filtered, and then 150 mL of the amino acid solution and 350 mL of the sugar solution were filled into different chambers of a plastic container partitioned with a readily-releasable seal, the chambers were sealed, and high-pressure steam sterilization was carried out under a nitrogen atmosphere. After cooling and drying, it was packaged under nitrogen gas, together with an iron-based oxygen absorber (trade name: "AGELESS^{™} ZH"), using a gas impermeable outer packaging material (product of Dai Nippon Printing Co., Ltd.) having an aluminum foil barrier layer, to obtain an infusion solution for peripheral intravenous administration.
The titratable acidity of the sugar solution was 3, and the titratable acidity of the infusion after mixing the sugar solution and amino acid solution was 7.

### [Example 2]

An infusion solution for peripheral intravenous administration was obtained in the same manner as Example 1, except that sodium hydrogen sulfite concentration in the amino acid solution formulation was changed to 0.1 g/L.

### [Example 3]

An infusion solution for peripheral intravenous administration was obtained in the same manner as Example 1, except that sodium hydrogen sulfite concentration in the amino acid solution formulation was changed to 0.2 g/L.

### [Example 4]

The formulation for the 50% sodium lactate solution in the amino acid solution formulation of Example 1 was changed to 13.1 g/L. An infusion solution for peripheral intravenous administration was obtained in the same manner as Example 1, except that the 50% sodium lactate solution was also added at 0.91 g/L to a pH of 4.5 in the formulation of the sugar solution.

### [Example 5]

The formulation for the 50% sodium lactate solution in the amino acid formulation of Example 1 was changed to 9.0 g/L. An infusion solution for peripheral intravenous administration was obtained in the same manner as Example 1, except that the 50% sodium lactate solution was also added at 2.7 g/L to a pH of 4.7 in the formulation of the sugar solution.

### [Reference Example 1]

The 50% sodium lactate solution was omitted from the amino acid solution formulation and the sodium hydrogen sulfite concentration was changed to 0.3 g/L, for a pH of 8.0. An infusion solution for peripheral intravenous administration was obtained in the same manner as Example 1, except that the 50% sodium lactate solution was also added at 6.5 g/L in the formulation of the sugar solution.

### [Test 1]

The L-cysteine contents of the infusion solutions for peripheral intravenous administration of Example 1, Example 2, Example 3 and Reference Example 1 after sterilization were measured. The contents are listed in Table 1, with 100% as the amount of L-cysteine added.

**[Table 1]**

| | Amino acid solution pH | Sodium hydrogen sulfite concentration g/L | L-Cys content% |
|---|---|---|---|
| Example 1 | 7.0 | 0.05 | 92.2 |
| Example 2 | 7.0 | 0.1 | 92.4 |
| Example 3 | 6.9 | 0.2 | 94.4 |
| Reference Example 1 | 8.0 | 0.3 | 90.3 |

The results demonstrated that when the pH of the amino acid solution is neutral, decomposition of L-cysteine during sterilization can be prevented by adding sodium hydrogen sulfite at 0.05-0.2 g/L.

### [Test 2]

The infusion solutions for peripheral intravenous administration of Example 1, Example 2, Example 3 and Reference Example 1 were subjected to prolonged storage at 40°C, 75% relative humidity, and the L-cysteine contents of the amino acid solutions were measured. The contents are listed in Table 2, with 100% as the amount of L-cysteine added.

**[Table 2]**

| | Number of months stored | | | | |
|---|---|---|---|---|---|
| | 0 | 1 | 2 | 4 | 6 |
| Example 1 | 92.2 | 91.0 | 90.5 | 88.8 | 87.9 |
| Example 2 | 92.4 | 91.7 | 90.4 | 89.7 | 88.2 |
| Example 3 | 94.4 | 92.6 | 91.7 | 89.0 | 88.2 |
| Reference Example 1 | 90.3 | 90.0 | 88.1 | 85.6 | 81.6 |

The results demonstrated that L-cysteine is stable with prolonged storage with addition of sodium hydrogen sulfite at 0.05-0.2 g/L as in Example 1, Example 2 and Example 3.

### [Test 3]

The infusion solutions for peripheral intravenous administration of Example 1 and Reference Example 1 were subjected to prolonged storage at 40°C, 75% relative humidity, and the vitamin B1 contents in the sugar solutions were measured. Table 3 shows the contents, with 100% as the amount of vitamin B1 added.

**[Table 3]**

| | Number of months stored | | | |
|---|---|---|---|---|
| | 0 | 1 | 3 | 6 |
| Example 1 | 85.0 | 83.3 | 77.7 | 75.1 |
| Reference Example 1 | 87.8 | 84.2 | 76.3 | 71.2 |

The results demonstrated that vitamin B1 was stable during prolonged storage in Example 1 and Reference Example 1.

### [Test 4]

For the infusion solutions for peripheral intravenous administration of Example 1, Example 2, Example 3 and Reference Example 1, the sugar solutions and amino acid solutions were mixed and the obtained infusions were allowed to stand at room temperature and under diffused light, with periodical measurement of the vitamin B1 contents of the infusions. Table 4 shows the vitamin B1 contents, with 100% as the contents immediately after admixture.

**[Table 4]**

| | Sodium hydrogen sulfite concentration (g/L) | Hour after mixing | | | |
|---|---|---|---|---|---|
| | | 0 | 5 | 10 | 15 |
| Example 1 | 0.05 | 100.0 | 99.4 | 98.4 | 98.6 |
| Example 2 | 0.1 | 100.0 | 99.9 | 98.2 | 95.5 |
| Example 3 | 0.2 | 100.0 | 97.5 | 94.2 | 92.1 |
| Reference Example 1 | 0.3 | 100.0 | 93.0 | 91.5 | 88.1 |

The results demonstrated that Example 1, Example 2 and Example 3. in which the sodium hydrogen sulfite addition was reduced, had improved stability of vitamin B1 compared to Reference Example 1.

### [Test 5]

The infusion solutions for peripheral intravenous administration of Example 1, Example 4 and Example 5 were stored at room temperature for 1 month, and the vitamin B1 contents in the sugar solutions were measured. Table 5 shows the contents, with 100% as the amount of vitamin B1 added.

**[Table 5]**

| | Sugar solution | | Amino acid solution pH | Vitamin B1 content% |
|---|---|---|---|---|
| | pH | Titratable acidity | | |
| Example 1 | 4.3 | 3 | 7.0 | 92.3 |
| Example 4 | 4.5 | 3 | 7.0 | 91.8 |
| Example 5 | 4.7 | 3 | 7.0 | 91.4 |

The results demonstrated that the titratable acidity and amino acid solution pH did not change even at pH 4.7, which exceeds the value of pH 4.5 as the maximum pH for vitamin B1 parenteral solutions according to the Japanese Pharmacopeia, and that vitamin B1 was also stable.

### [Industrial Applicability]

According to the infusion solution for peripheral intravenous administration of the invention it is possible, with a small amount of acid, to keep the sugar solution at pH 4.0-4.7 which is the pH range in which vitamin B1 is stable and, with a small amount of sodium hydrogen sulfite, to keep the amino acid solution at near pH 7 to allow stabilization of amino acids.

## Claims

1. An infusion solution for peripheral intravenous administration comprising a sugar solution containing sugars, a portion of electrolytes and vitamin B1, and an amino acid solution containing amino acids, a portion of electrolytes and a sulfite, separately housed in a multichamber vessel constructed in a connectable manner, wherein (1) the sugar solution contains calcium gluconate, contains no greater than 1.4 g/L sodium lactate, has a pH of 4.0-4.7 and has a titratable acidity of 2-4, (2) the amino acid solution contains 4-15 g/L sodium lactate, contains no calcium gluconate, has a pH of 6.8-7.2, contains free L-cysteine as cysteine and contains 0.05-0.2 g/L sodium hydrogen sulfite, and (3) after the sugar solution and amino acid solution have been mixed, the infusion solution for peripheral intravenous administration has an infusion pH of 6.5-7.1 and a titratable acidity of 5-10.

2. An infusion solution for peripheral intravenous administration comprising a sugar solution containing sugars, a portion of electrolytes and vitamin B1, and an amino acid solution containing amino acids, a portion of electrolytes and a sulfite, separately housed in a multichamber vessel with a connectable construction, wherein (1) the sugar solution contains calcium gluconate, contains no sodium lactate, has a pH of 4.0-4.5 and has a titratable acidity of 2-4, (2) the amino acid solution contains sodium lactate, contains no calcium gluconate, has a pH of 6.8-7.2, contains free L-cysteine as cysteine and contains 0.05-0.2 g/L sodium hydrogen sulfite, and (3) after the sugar solution and amino acid have been mixed, the infusion solution for peripheral intravenous administration has a pH of 6.5-7.1 and a titratable acidity of 5-10.

3. An infusion solution for peripheral intravenous administration according to claim 1 or 2, wherein the sugar solution contains 50-200 g/L glucose, 1-5 mg/L vitamin B1 as thiamine, 0-1.4 g/L sodium lactate, 0.5-2 g/L sodium chloride, 1-3 g/L calcium gluconate, 0.5-2 g/L magnesium sulfate hydrate and 1-4 mg/L zinc sulfate, the amino acid solution contains 50-200 g/L amino acids as free amino acids, 4-15 g/L sodium lactate, 3-12 g/L dipotassium phosphate and 0.05-0.2 g/L sodium hydrogen sulfite, and the volume ratio of the sugar solution and the amino acid solution is 2-3:1.

4. An infusion solution for peripheral intravenous administration according to any one of claims 1 to 3,
wherein the amino acid composition of the amino acid solution, as free amino acids, is: 10-20 g/L L-leucine, 6-12 g/L L-isoleucine, 6-12 g/L L-valine, 8-16 g/L L-lysine, 2-10 g/L L-threonine, 1-3 g/L L-tryptophan, 2-6 g/L L-methionine, 0.5-2 g/L L-cysteine, 4-10 g/L L-phenylalanine, 0.3-1 g/L L-tyrosine, 7-16 g/L L-arginine, 3-8 g/L L-histidine, 5-12 g/L L-alanine, 3-8 g/L L-proline, 2-5 g/L L-serine, 4-9 g/L glycine, 0.5-2 g/L L-aspartic acid, 0.5-2 g/L L-glutamic acid.

5. An infusion solution for peripheral intravenous administration according to any one of claims 1 to 4,
wherein the electrolyte composition of the sugar solution is Na⁺: 9-47 mEq/L, Ca²⁺: 5-20 mEq/L, Mg²⁺: 5-20 mEq/L, Zn²⁺: 5-20 µmol/L, Cl⁻: 5-35mEq/L, the electrolyte composition of the amino acid solution is Na⁺: 35-150 mEq/L, K⁺: 30-140 mEq/L, phosphorus: 15-70 mmol/L, and the volume ratio of the sugar solution and amino acid solution is 2-3:1.

6. An infusion solution for peripheral intravenous administration according to any one of claims 1 to 5,
wherein (1) to the sugar solution there are added 50-200 g/L glucose, 1-5 mg/L vitamin B1 as thiamine, 0.5-2 g/L sodium chloride, 1-3 g/L calcium gluconate, 0.5-2 g/L magnesium sulfate and 1-4 mg/L zinc sulfate, the pH of the sugar solution is 4.0-4.5 and the titratable acidity of the sugar solution is 2-4, (2) to the amino acid solution there are added, as free amino acids, 10-20 g/L L-leucine, 6-12 g/L L-isoleucine, 6-12 g/L L-valine, 8-16 g/L L-lysine, 2-10 g/L L-threonine, 1-3 g/L L-tryptophan, 2-6 g/L L-methionine, 0.5-2 g/L L-cysteine, 4-10 g/L L-phenylalanine, 0.3-1 g/L L-tyrosine, 7-16 g/L L-arginine, 3-8 g/L L-histidine, 5-12 g/L L-alanine, 3-8 g/L L-proline, 2-5 g/L L-serine, 4-9 g/L glycine, 0.5-2 g/L L-aspartic acid and 0.5-2 g/L L-glutamic acid, with 4-15 g/L sodium lactate, 3-12 g/L dipotassium phosphate and 0.05-0.2 g/L sodium hydrogen sulfite, L-cysteine is added as free L-cysteine, and the pH of the amino acid solution is 6.8-7.2, and (3) the volume ratio of the sugar solution and amino acid solution is 7:3, and after the sugar solution and amino acid solution have been mixed, the pH of the infusion is 6.5-7.1 and the titratable acidity is 5-10.
